(19) Europäisches Patentamt
European Patent Office
Office européen des brevets

(11) Veröffentlichungsnummer : **0 166 173**
**B1**

(12) # EUROPÄISCHE PATENTSCHRIFT

(45) Veröffentlichungstag der Patentschrift : **02.03.88**

(51) Int. Cl.⁴ : **C 07 D 229/00**, C 08 G 18/79

(21) Anmeldenummer : **85106053.3**

(22) Anmeldetag : **17.05.85**

(54) **Verfahren zur Herstellung von Uretdiongruppen aufweisenden Polyisocyanaten.**

(30) Priorität : **30.05.84 DE 3420114**

(43) Veröffentlichungstag der Anmeldung : **02.01.86 Patentblatt 86/01**

(45) Bekanntmachung des Hinweises auf die Patenterteilung : **02.03.88 Patentblatt 88/09**

(84) Benannte Vertragsstaaten : **DE FR GB IT NL**

(56) Entgegenhaltungen :
**EP-A- 0 099 376**
**CA-A- 894 292**
**DE-A- 1 670 720**
**GB-A- 1 200 432**
**GB-A- 2 113 673**
**US-A- 3 108 100**
**CHEMISCHE BERICHTE, Band 110, Nr. 3, 1977, Seiten 1130-1139, Weinheim, DE; N. KUHN et al.: "1,3-Dialkyldiazetidin-2,4-dione.Struktur und Molekülspektren"**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Scholl, Hans-Joachim, Dr.**
**Rudolf-Sohm-Strasse 28**
**D-5000 Koeln 60 (DE)**

**EP 0 166 173 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Herstellung von Uretdiongruppen aufweisenden Polyisocyanaten durch teilweise Dimerisierung der Isocyanatgruppen von organischen Diisocyanaten mit aliphatisch und/oder cycloaliphatisch gebundenen Isocyanatgruppen unter Verwendung von Antimon (V) fluorid als Dimerisierungskatalysator.

Verfahren zur Herstellung von Uretdionen durch Dimerisierung von aliphatischen Isocyanaten sind bereits bekannt. Gemäß DE-OS 1 670 720 werden hierzu Phosphine oder Bortrifluorid als Dimerisierungskatalysator verwendet. Ein Nachteil dieser Katalysatoren ist die Bildung von beträchtlichen Mengen an Isocyanuraten als Nebenprodukte bei der Dimerisierungsreaktion. Gemäß DE-OS 3 227 779 werden zur Dimerisierung von ganz speziellen Ausgangsdiisocyanaten (Diisocyanatohexane mit werzweigter Kohlenstoffkette) spezielle Aminophosphine wie z. B. Tris-(dimethylamino)-phosphin als Dimerisierungskatalysator eingesetzt, die, der Aussage dieser Veröffentlichung folgend, die Dimerisierung dieser speziellen Ausgangsdiisocyanate bei wesentlich verbesserter Ausbeute an Uretdiongruppen gestatten. Die Herstellung von Uretdiongruppen aufweisenden Polyisocyanaten auf Basis anderer aliphatischer bzw. cycloaliphatischer Diisocyanate als den genannten sehr speziellen Verbindungen ist in dieser Vorveröffentlichung nicht beschrieben.

Es war daher die der Erfindung zugrundeliegende Aufgabe, ein neues Verfahren zur Verfügung zu stellen, welches die Dimerisierung beliebiger organischer Diisocyanate mit aliphatisch und/oder cycloaliphatisch gebundenen Isocyanatgruppen in hohen Ausbeuten ohne die Bildung von unerwünschten Nebenprodukten gestattet.

Diese Aufgabe konnte mit dem nachstehend näher beschriebenen erfindungsgemäßen Verfahren gelöst werden.

Gegenstand der Erfindung ist ein Verfahren zur Herstellung von Uretdiongruppen aufweisenden Polyisocyanaten mit aliphatisch und/oder cycloaliphatisch gebundenen Isocyanatgruppen durch Dimerisierung eines Teils der Isocyanatgruppen von organischen Diisocyanaten mit aliphatisch und/oder cycloaliphatisch gebundenen Isocyanatgruppen in Gegenwart eines, die Dimerisierung beschleunigenden, Katalysators und Abbruch der Dimerisierungsreaktion beim jeweils erwünschten Dimerisierungsgrad durch Zugabe eines Katalysatorengifts oder destillativer Entfernung des Katalysators, dadurch gekennzeichnet, daß man als Katalysator Antimon (V) fluorid verwendet.

Das erfindungsgemäße Verfahren eignet sich zur Teildimerisierung beliebiger organischer Diisocyanate mit aliphatisch und/oder cycloaliphatisch gebundenen Isocyanatgruppen. Für das erfindungsgemäße Verfahren geeignete Ausgangsdiisocyanate sind insbesondere aliphatische oder cycloaliphatische Diisocyanate eines über 139, vorzugsweise von 140 bis 250 liegenden Molekulargewichts wie z. B. Tetramethylendiisocyanat, Hexamethylendiisocyanat, Dodecanmethylendiisocyanat, Cyclobutan-1,3-diisocyanat, Cyclohexan-1,3- und -1,4-diisocyanat, 1-Isocyanato-3,3,5-trimethyl-5-isocyanatomethyl-cyclohexan (IPDI), Perhydro-2,4- und/oder -2,6-diisocyanato-toluol oder Perhydro-2,4'- und/oder -4,4'-diisocyanato-diphenylmethan oder beliebige Gemische dieser Diisocyanate.

Besonders bevorzugt wird 1,6-Diisocyanatohexan (Hexamethylendiisocyanat) als Ausgangsdiisocyanat eingesetzt.

Das erfindungsgemäße Verfahren wird vorzugsweise lösungsmittelfrei innerhalb des Temperaturbereichs von 0 bis 80 °C, insbesondere von 10 bis 50 °C durchgeführt.

Der erfindungswesentliche Katalysator, Antimon (V) fluorid, stellt eine bekannte Chemikalie dar und ist beispielsweise in « Gmelins Handbuch der anorganischen Chemie », 8. Auflage, Systemnummer 18 (1949), Seite 401 beschrieben. Der Katalysator wird in einer Menge von 0,2 bis 5 Gew.-%, vorzugsweise 0,5 bis 2 Gew.-%, bezogen auf Ausgangsdiisocyanat eingesetzt.

Die Dimerisierungsreaktion wird vorzugsweise innerhalb der genannten Temperaturbereiche bis zum Erreichen eines Dimerisierungsgrads von 5 bis 35, vorzugsweise 10 bis 25 % durchgeführt. Unter « Dimerisierungsgrad » ist hierbei der Prozentsatz der Isocyanatgruppen im Ausgangsdiisocyanat zu verstehen, die in Uretdiongruppen überführt werden. Der Dimerisierungsgrad kann während der Dimerisierungsreaktion beispielsweise durch fortlaufende Bestimmung des Brechungsindex oder des NCO-Gehalts des Reaktionsansatzes verfolgt werden.

Nach Erreichen des gewünschten Dimerisierungsgrades wird die Dimerisierungsreaktion vorzugsweise durch Zugabe eines Katalysatorengifts abgebrochen. Der Abbruch der Dimerisierungsreaktion kann jedoch auch durch destillative Entfernung des Dimerisierungskatalysators, beispielsweise zusammen mit überschüssigem, nicht umgesetztem Ausgangsdiisocyanat erfolgen. Im allgemeinen wird, abhängig von der Katalysatormenge und der Reaktionstemperatur, der angestrebte Dimerisierungsgrad innerhalb der obengenannten Bereiche nach einem Zeitraum von 3 bis 72 Stunden erreicht.

Geeignete Katalysatorengifte sind alle beliebigen Verbindungen, die, wie in der obengenannten Literaturstelle angegeben, mit Antimon (V) fluorid chemische Umsetzungen eingehen oder Antimon (V) fluorid an ihrer Oberfläche adsorptiv binden. Hierbei evtl. entstehende Feststoffanteile können beispielsweise durch Filtration aus dem Reaktionsgemisch entfernt werden. Geeignete Katalysatorengifte sind beispielsweise beliebige organische Carbonsäuren, d. h. beliebige organische Verbindungen, die mindestens eine freie Carboxylgruppe aufweisen wie z. B. Weinsäure, Phthalsäure, Benzoesäure oder

das Mono-Kaliumsalz der Weinsäure, Zinkstaub oder Schwefel. Besonders bevorzugt wird das Mono-Kaliumsalz der Weinsäure als Katalysatorgift verwendet. Die Menge des Katalysatorgifts wird vorzugsweise so bemessen, daß es zur vollständigen « Neutralisation » des Katalysators ausreicht. Diese Menge kann beispielsweise durch einen orientierenden Vorversuch ermittelt werden.

Die Verfahrensprodukte können gewünschtenfalls im Anschluß an die Dimerisierungsreaktion von nicht umgesetztem Ausgangsdiisocyanat bis zu einem Restgehalt von weniger als 1 %, bezogen auf Gesamtgemisch, durch Dünnschichtdestillation befreit werden. Wie bereits angedeutet, kann diese destillative Entfernung von nicht umgesetztem Ausgangsdiisocyanat auch mit dem Abbruch der Dimerisierungsreaktion durch destillative Entfernung des Katalysators kombiniert werden. Diese Art der Beendigung der Dimerisierungsreaktion ist jedoch im Vergleich zur Zugabe eines Katalysatorengifts weniger bevorzugt.

Die erfindungsgemäßen Verfahrensprodukte stellen auch nach Entfernung des überschüssigen Ausgangsdiisocyanats bei Raumtemperatur niedrigviskose Substanzen dar, die laut IR-spektroskopischem Befund keine Isocyanuratgruppen aufweisen. Der NCO-Gehalt liegt nur geringfügig (bis max. 20 %) unterhalb des theoretischen Werts, der sich aus der Formel

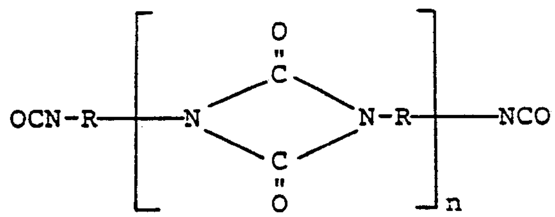

mit n = 1 errechnet. In dieser Formel steht R für den indifferenten Rest des Ausgangsdiisocyanats $R(NCO)_2$. Diese geringfügige Abweichung vom theoretischen Wert ist auf das Vorliegen von oligomeren Uretdiondiisocyanaten (n = ganze Zahl größer als 1, vorzugsweise 2 bis 5) zurückzuführen.

Die erfindungsgemäßen Verfahrensprodukte können selbstverständlich in an sich bekannter Weise mit geeigneten Blockierungsmitteln für Isocyanatgruppen wie z. B. Phenol, ε-Caprolactam, Malonsäurediethylester oder Acetessigsäureethylester blockiert werden.

Die erfindungsgemäßen Verfahrensprodukte bzw. ihre durch die genannte Blockierungsreaktion erhaltenen Derivate stellen wertvolle Ausgangsmaterialien zur Herstellung von Polyurethankunststoffen nach dem Isocyanat-Polyadditionsverfahren dar. Sie eignen sich insbesondere als Isocyanatkomponente in Zweikomponentenpolyurethanlacken. Wegen des Vorliegens von Uretdiongruppen, die als verkappte Isocyanatgruppen aufzufassen sind, eignen sich die erfindungsgemäßen Verfahrensprodukte insbesondere zur Herstellung von Hitze-vernetzbaren Beschichtungen.

Die folgenden Beispiele erläutern die Erfindung. Alle Prozentangaben betreffen, soweit nicht anderslautend ausgeführt, Gewichtsprozente. Alle Angaben in « Teilen » betreffen Gewichtsteile.

### Beispiel 1

100 Teile Hexamethylendiisocyanat und 1 Teil Antimon (V) fluorid werden 40 Stunden bei Raumtemperatur gerührt, danach ist der NCO-Gehalt auf 41,9 % gefallen. Die Reaktion wird durch Zugabe von 5 Teilen Zinkpulver gestoppt. Man rührt 2 Stunden nach, filtriert ab und erhält nach Dünnschichtdestillation 31 Teile Isocyanatouretdion mit den Daten :

NCO-Gehalt 22,3 %, freies Hexamethylendiisocyanat : < 0,6 %, Viskosität (23 °C) : 70 mPa · s.

### Beispiel 2

100 Teile Hexamethylendiisocyanat und 0,5 Teile Antimon(V) fluorid werden 5 Stunden bei 50 °C gerührt. Danach ist der Brechungsindex auf $n_D^{24\,°C}$ : 1,461 0 (Anfangswert = $n_D^{21\,°C}$ : 1,453 0) gestiegen und der NCO-Gehalt auf 43 % abgesunken. Nach Dünnschichtdestillation erhält man 22 Teile Isocyanatouretdion mit den Daten :

NCO-Gehalt 22,0 %, freies Hexamethylendiisocyanat : < 1 %, Viskosität (23 °C) : 60 mPa · s.

### Beispiel 3

100 Teile Hexamethylendiisocyanat und 0,8 Teile Antimon (V) fluorid werden 60 h bei 20 °C stehen gelassen. Danach ist der Brechungsindex $n_D^{23\,°C}$ : 1,465 0 gestiegen und der NCO-Gehalt beträgt 39,7 %. Die Reaktion wird durch Zugabe von 2 Teilen Weinsäure-Monokaliumsalz gestoppt. Man rührt 2 h nach, filtriert ab und erhält nach Dünnschichtdestillation 35 Teile Isocyanatouretdion mit den Daten :

NCO-Gehalt 22,5 % freies Hexamethylendiisocyanat < 0,6 %, Viskosität 70 mPa · s.

**Patentansprüche**

1. Verfahren zur Herstellung von Uretdiongruppen aufweisenden Polyisocyanaten mit aliphatisch und/oder cycloaliphatisch gebundenen Isocyanatgruppen durch Dimerisierung eines Teils der Isocyanatgruppen von organischen Diisocyanaten mit aliphatisch und/oder cycloaliphatisch gebundenen Isocyanatgruppen in Gegenwart eines, die Dimerisierung beschleunigenden, Katalysators und Abbruch der Dimerisierungsreaktion beim jeweils erwünschten Dimerisierungsgrad durch Zugabe eines Katalysatorengifts oder destillativer Entfernung des Katalysators, dadurch gekennzeichnet, daß man als Katalysator Antimon (V) fluorid verwendet.

2. Verfahren gemäß Anspruch 1, dadurch gekennzeichnet, daß man als Ausgangsdiisocyanat 1,6-Diisocyanatohexan verwendet.

3. Verfahren gemäß Anspruch 1 und 2, dadurch gekennzeichnet, daß man nach Beendigung der Dimerisierungsreaktion nicht umgesetztes Ausgangsdiisocyanat durch Dünnschichtdestillation entfernt.

4. Verfahren gemäß Anspruch 1 bis 3, dadurch gekennzeichnet, daß man als Katalysatorengift organische Carbonsäuren, Zinkstaub oder Schwefel verwendet.

**Claims**

1. A process for the production of uretdione polyisocyanates containing aliphatically and/or cycloaliphatically bound isocyanate groups by dimerization of some of the isocyanate groups of organic diisocyanates containing aliphatically and/or cycloaliphatically bound isocyanate groups in the presence of a catalyst which accelerates the dimerization reaction and termination of the dimerization reaction at the particular degree of dimerization required by addition of a catalyst poison or by removal of the catalyst by distillation, characterized in that antimony (V) fluoride is used as the catalyst.

2. A process as claimed in Claim 1, characterized in that 1,6-diisocyanatohexane is used as the starting diisocyanate.

3. A process as claimed in Claims 1 and 2, characterized in that unreacted starting diisocyanate is removed by thinlayer distillation on completion of the dimerization reaction.

4. A process as claimed in Claims 1 to 3, characterized in that organic carboxylic acids, zinc dust or sulfur is used as the catalyst poison.

**Revendications**

1. Procédé pour la fabrication de polyisocyanates à groupes uretdiones ayant des groupes isocyanates à liaison aliphatique et/ou à liaison cycloaliphatique par dimérisation d'une partie des groupes isocyanates de diisocyanates organiques ayant des groupes isocyanates à liaison aliphatique et/ou à liaison cycloaliphatique en présence d'un catalyseur accélérant la dimérisation et interruption de la réaction de dimérisation chaque fois au degré de dimérisation souhaité par addition d'un poison du catalyseur ou élimination du catalyseur par distillation, caractérisé en ce que l'on utilise comme catalyseur le fluorure d'antimoine (V).

2. Procédé selon la revendication 1, caractérisé en ce que l'on utilise comme diisocyanate de départ le 1,6-diisocyanatohexane.

3. Procédé selon la revendication 1 ou 2, caractérisé en ce que l'on élimine le diisocyanate de départ non transformé par distillation en couche mince après la fin de la réaction de dimérisation.

4. Procédé selon l'une des revendications 1 à 3, caractérisé en ce que l'on utilise comme poison du catalyseur des acides carboxyliques organiques, de la poudre de zinc ou du soufre.